# EUROPÄISCHE PATENTSCHRIFT

(11) **EP 0 959 956 B1**
(45) Veröffentlichungstag und Bekanntmachung des Hinweises auf die Patenterteilung: **12.12.2001**
(21) Anmeldenummer: 98907837.3
(22) Anmeldetag: 21.01.1998
(51) Int. Cl.: A63B 21/00

(54) **PATIENTENÜBERWACHUNGSSYSTEM**
PATIENT MONITORING SYSTEM
SYSTEME DE SURVEILLANCE DE PATIENT

(30) Priorität: 22.01.1997 DE 19702150
(43) Veröffentlichungstag der Anmeldung: 01.12.1999
(73) Patentinhaber: SIEMENS AKTIENGESELLSCHAFT, 80333 München (DE)
(72) Erfinder: ABRAHAM-FUCHS, Klaus, D-91058 Erlangen (DE); BIRKHÖLZER, Thomas, D-91085 Weisendorf (DE); HEROLD, Alexander, D-91052 Erlangen (DE)
(86) Internationale Anmeldenummer: DE9800175
(87) Internationale Veröffentlichungsnummer: WO9832497

(56) Entgegenhaltungen:
- WO-A-91/12786
- US-A- 5 538 486

## Beschreibung

Viele orthopädische Störungen oder Krankheiten werden durch fehlerhafte Bewegungsabläufe oder Körperhaltungen verursacht oder verschlimmert (z.B. Rückenprobleme). Die Vorbeugung bzw. Therapie besteht zu einem großen Teil in der Einübung verbesserter Bewegungsabläufe oder Körperhaltungen. Dabei ist es für den Erfolg wesentlich, die entsprechenden Bewegungsabläufe oder Körperhaltungen richtig einzuüben, da sonst sogar eine Verschlechterung des Gesundheitszustandes eintreten kann.

Heute werden unter Aufsicht (Krankengymnastik) Bewegungs- oder Belastungsabläufe trainiert bzw. entsprechende Übungen geschult. Zu Hause werden dann die Übungen ohne Aufsicht nachvollzogen.

In DE 40 39 648 A1 ist ein Meßwertverarbeitungssystem für ein biologisches Objekt beschrieben, bei dem Sensoren für Meßwerte vorhanden sind. Die Meßwerte werden mathematisch bewertet und mit vorgegebenen Meßwertstrukturen verglichen. In Abhängigkeit davon kann eine Speichereinrichtung, eine Steuereinrichtung oder eine Alarmeinrichtung getriggert werden. Hinsichtlich der Ausbildung für die orthopädische Überwachung und die Erfassung von Bewegungen des Objekts läßt sich diesen Stand der Technik nichts entnehmen. Dies gilt sinngemäß auch für das in DE 32 09 850 C2 beschriebene Auswerteverfahren für ein EKG, bei dem EKG-Elektroden als Sensoren vorgesehen sind, die für die unmittelbare Erfassung von Bewegungen nicht geeignet sind.

In der Druckschrift WO 91/12786 ist eine orthopädische Übungsvorrichtung beschrieben, welche ein zwei relativ zueinander bewegliche Teilabschnitte umfassendes Gehäuse aufweist.

Die Teilabschnitte weisen jeweils zwei einander gegenüberliegende, parallel zueinander ausgerichtete Stangen auf, zwischen denen ein Körperteil gebracht werden kann. Die zwei Stangen des einen Teilabschnittes des Gehäuses sind über ein hinsichtlich seiner Bewegungsmöglichkeiten einstellbares Scharnier mit den zwei anderen Stangen des zweiten Teilabschnittes verbunden. Wird beispielsweise ein Bein eines Patienten in die Übungsvorrichtung eingeführt, wobei der Unterschenkel in dem einen und der Oberschenkel in dem anderen Teilabschnitt des Gehäuses eingespannt wird, kann der Patient im Rahmen der an dem Scharnier einstellbaren Bewegungsfreiheit den Unterschenkel relativ zu dem Oberschenkel bewegen. An den Stäben der Teilabschnitte der Übungsvorrichtung sind Drucksensoren angeordnet, welche bei Übungen eines Patienten auftretende Druckkräfte auf die Stäbe der Übungsvorrichtung messen.

Aus der US 5,538,486 ist außerdem eine zum Trainieren von Muskelgruppen vorgesehene Therapievorrichtung bekannt, welche eine dehnbare Schnur aufweist, die mit einer Sensoranordnung verbunden ist. Die Sensoranordnung ist ihrerseits einem Mikroprozessorkontrollmodul fest zugeordnet, welches in einem Gehäuse aufgenommen ist. Das Gehäuse ist mit einem Griff verbunden, der zur Betätigung der Therapievorrichtung vorgesehen ist. Zur Durchführung von Übungen ist das freie Ende der dehnbaren Schnur an einer geeigneten Vorrichtung befestigt, so daß ein Patient durch Ziehen an dem Griff Kraftübungen mit der Therapievorrichtung ausführen kann. Dabei werden mit der mit der dehnbaren Schnur zusammenwirkenden Sensoranordnung Sensorsignale gemessen, welche von dem Mikroprozessorkontrollmodul ausgewertet und als Kraftwerte auf einem Display angezeigt, in einem Speicher gespeichert bzw. mit vorgegebenen Kraftwerten verglichen werden können.

Der Erfindung liegt die Aufgabe zugrunde, ein orthopädisches Patientenüberwachungssystem zu schaffen, das für den Gebrauch durch den medizinischen Laien in einer gewohnten Umgebung geeignet ist und ihm Informationen über die Ausführung der Übungen gibt.

Diese Aufgabe ist erfindungsgemäß gelöst durch die Merkmale des Patentanspruchs 1. Das erfindungsgemäße Überwachungssystem eignet sich insbesondere zur Kontrolle von Bewegungen und Bewegungsabläufen bei orthopädischen Störungen oder Krankheiten.

Mit Hilfe von geeigneten Sensoren werden Druck- und Scherkräfte (z.B. mit piezoelektrischen Folien) und/oder Beschleunigungen gemessen. Die Messung der Druck- und Scherkräfte kann z.B. durch in den Sohlen von Schuhen eingelassenene Sensoren erfolgen. Meßwerte können dabei sowohl Einzelmessungen als auch dynamischen Sequenzen entsprechen. Dies kann entweder in einer Trainingssituation (regelmäßige Übungen) oder im Alltagsgebrauch (z.B. Kontrolle der Sitzhaltung) erfolgen. Die Meßwerte können entweder direkt in einer am Körper getragenen Einheit ausgewertet werden (Übertragung durch Kabel oder Telemetrie) oder per Telemetrie an eine stationäre Einheit übertragen werden.

Die Meßwerte werden mit vorgegebenen, gespeicherten Idealwerten verglichen. Die Idealwerte sind aus den vom Experten (Arzt oder Krankengymnast) vorgegebenen idealen Bewegungsabläufen oder Körperhaltungen abgeleitet. Die Eingabe kann dabei durch direkte Vorgabe von bestimmten Mustern oder Werten erfolgen, durch Auswahl aus einer gespeicherten Menge von Vorgaben oder durch Training, d.h. durch Messung und Speicherung einer realen Situation (z.B. Vormachen des Therapeuten, Übung unter Aufsicht und Speicherung einer geglückten Übung).

Die Beurteilung der Meßwerte kann auch selbstlernend (z.B. mit neuronalen Netzen) erfolgen, z.B. indem der Benutzer bei einem falschen Bewegungsablauf oder einer falschen Körperhaltung (z.B. spürbar durch Schmerz) dem Gerät eine Markierung gibt. Der dazu gehörige Datensatz einschließlich einer gewissen Vergangenheit (z.B. die letzten 30 s) wird gespeichert. Anhand der gespeicherten Datensätze lernt das System unerwünschte Zustände frühzeitig zu erkennen und kann den Benutzer rechtzeitig warnen, z.B. vor einer Schmerzempfindung.

Als weitere mögliche Zusatzfunktion können die aktuellen Meßwerte gespeichert werden (lokal oder in einer durch eine Netzwerkanbindung zugänglichen Datenbasis), um eine (spätere) Auswertung durch den Experten (Arzt oder Krankengymnast) zu ermöglichen. Dem Benutzer wird eine gezielte Rückmeldung gegeben, wie gut seine aktuellen Bewegungsabläufe oder Körperhaltungen mit den vorgegebenen Idealwerten übereinstimmen. Dazu können auch die Meßwerte mit Expertenwissen (lokal oder per Fernabfrage) kombiniert werden, um gezielte Handlungs- bzw. Verbesserungsanweisungen abzuleiten. Die Rückmeldung kann z.B. durch optische oder akustische Signale, durch Reizungen des Schmerz- oder Tastsinns (Biofeedback) oder durch komplexe Handlungsanweisungen erfolgen. Insbesondere ist auch eine telemetrische Übertragung von der Auswerteeinheit zu am Körper getragenen Einheiten (z.B. kleine Lautsprecher am Ohr) möglich.

Das Wesen der Erfindung liegt in der Kombination der oben beschriebenen Funktionen in einem Gerät für medizinische Laien, insbesondere die automatische Auswertung der Messungen und das Feedback an den Nutzer.

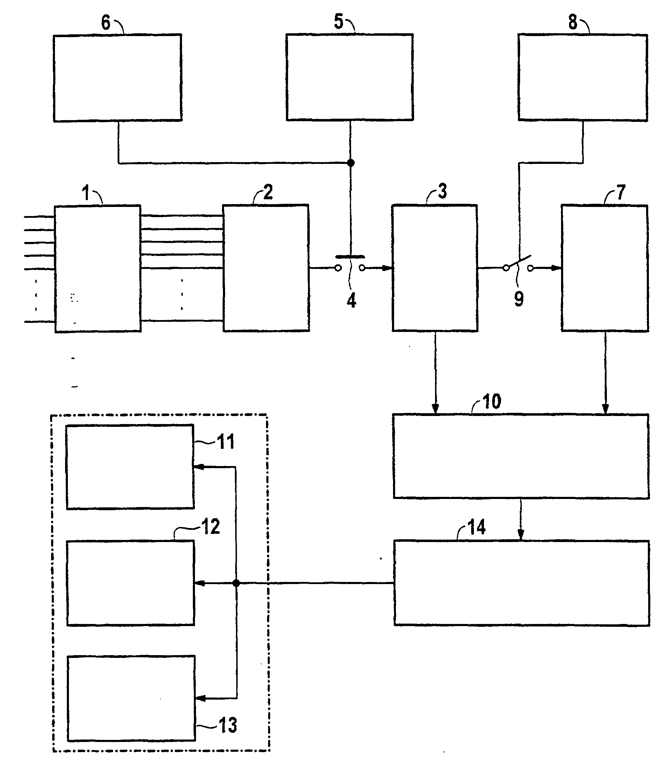

Die Erfindung ist nachfolgend anhand eines in der Zeichnung dargestellten Ausführungsbeispiels näher erläutert.

In der Zeichnung ist ein Sensorarray 1, z.B. mit Drucksensoren in der Schuhsohle, Muskelsensoren, Beschleunigungssensoren an einem bewegten Körperteil, dargestellt, dessen Meßwerte einer Signalvorverarbeitungseinheit 2 zugeführt werden. Die Signalvorverarbeitungseinheit 2, die die Meßwerte aller Sensoren vorverarbeitet, kann z.B. Filter, Verstärker, AD-Wandler und Mittel zur Signalverknüpfung und Parameterextraktion enthalten. Das Ausgangssignal der Signalvorverarbeitungseinheit 2 ist einem Speicher 3 über einen Schalter 4 zuführbar. Im Speicher 3 wird eine erste Signalmatrix der vorverarbeiteten Signale aller Sensoren im Sensorarray 1 in einem vorgegebenen Zeitfenster T1 - T2 als aktuelles Signalmuster gespeichert. Das Zeitfenster T1 - T2 wird durch ein Userinterface 5 vorgegeben, dem ein Trigger 6 zur Ein- und Ausschaltung zugeordnet ist, dessen Ausgangssignale aus aktuellen Sensorsignalen abgeleitet sind.

Dem Speicher 3 ist ein Speicher 7 für eine zweite Signalmatrix zugeordnet, die ein ideales Signalmuster verkörpert. Das ideale Signalmuster kann z.B. während einer Trainingsphase durch Mittelung mehrerer Signalmuster bestimmt werden. Die Einspeicherung des idealen Signalmusters wird durch ein Userinterface 8 gesteuert, das Schaltmittel 9 zur Einspeicherung dieses idealen Signalmusters betätigt, wenn der Speicher 1 ein solches ideales Signalmuster enthält.

Ein Komparator 10 vergleicht den Inhalt der Speicher 3 und 7 und liefert über eine Ausgabeeinheit 14 eine Information über das Vergleichsergebnis, die dem Benutzer bzw. Patienten über eine optische Anzeige 11, eine akustische Anzeige 12 oder ein Biofeedback 13 mitgeteilt werden kann. Das Biofeedback 13 kann z.B. in der Applikation eines Reizstroms an Muskel- oder Nervenzellen bestehen.

## Patentansprüche

1. Orthopädisches Patientenüberwachungssystem mit Bewegungssensoren (1) für biomedizinische Signale, die zur Messung von Druck- und Scherkräften und/oder Beschleunigungen ausgebildet sind, mit einem Speicher (3) für von diesen Signalen abgeleitete Informationen, einem Speicher (7) für ein vorgegebenes Informationsmuster, einem Komparator (10) für den Vergleich der Inhalte der beiden Speicher (3, 7) und Wiedergabemitteln (11, 12, 13) für das Vergleichsergebnis, **dadurch gekennzeichnet, daß** die Informationen des Speichers (7) für ein vorgegebenes Informationsmuster aus den Sensorsignalen gewonnen werden.

2. Orthopädisches Überwachungssystem nach Anspruch 1, bei dem ein Userinterface (8) vorhanden ist, durch das die Einspeicherung des vorgegebenen Informationsmusters in den zugehörigen Speicher (7) durch den Benutzer steuerbar ist.

3. Orthopädisches Überwachungssystem nach Anspruch 1 oder 2, bei dem ein Biofeedback (13) für Reizungen des Benutzers in Abhängigkeit vom Vergleichsergebnis vorgesehen ist.

4. Orthopädisches Überwachungssystem nach einem der Ansprüche 1 bis 3, bei dem die Sensoren (1) zur Abnahme von Signalen am Patienten ausgebildet sind, wobei die Sensoren in einem Kleidungsstück angeordnet oder einem Muskel oder bewegten Körperteil zugeordnet sind.

## Claims

1. Orthopaedic patient monitoring system having movement sensors (1) for biomedical signals, which are designed to measure compression forces and shearing forces and/or accelerations, having a memory (3) for information derived from these signals, a memory (7) for a predetermined information pattern, a comparator (10) for comparing the contents of the two memories (3, 7) and means (11, 12, 13) for delivering the comparison result, **characterised in that** the information in the memory (7) for a predetermined information pattern is obtained from the sensor signals.

2. Orthopaedic monitoring system according to Claim 1, in which there is a user interface (8) via which the storing of the predetermined information pattern in the associated memory (7) can be controlled by the user.

3. Orthopaedic monitoring system according to Claim 1 or 2, in which a biofeedback (13) is provided for stimulating the user as a function of the comparison result.

4. Orthopaedic monitoring system according to one of Claims 1 to 3, in which the sensors (1) are designed to take signals from the patient, the sensors being arranged in an article of clothing or being allocated to a muscle or to a moving body part.

## Revendications

1. Système orthopédique de surveillance de patient comprenant des détecteurs (1) de mouvements donnant des signaux biomédicaux qui sont conçus pour la mesure de forces de pression et de cisaillement et/ou d'accélération, une mémoire (3) pour des informations déduites de ces signaux, une mémoire (7) pour un modèle d'informations données à l'avance, un comparateur (10) pour la comparaison des contenus des deux mémoires (3, 7) et des moyens (11, 12, 13) de restitution du résultat de la comparaison, **caractérisé en ce que** les informations de la mémoire (7) pour un modèle d'informations données à l'avance sont obtenues à partir des signaux de détecteur.

2. Système orthopédique de surveillance suivant la revendication 1, dans lequel il est prévu une interface (8) utilisateur par laquelle la mémorisation du modèle d'informations données à l'avance dans la mémoire (7) associée peut être commandée par l'utilisateur.

3. Système orthopédique de surveillance suivant la revendication 1 ou 2, dans lequel il est prévu un biofeedback (13) pour des excitations de l'utilisateur en fonction du résultat de la comparaison.

4. Système orthopédique de surveillance suivant l'une des revendications 1 à 3, dans lequel les détecteurs sont conçus pour prélever des signaux sur le patient, les détecteurs étant disposés dans une pièce de vêtement ou associés à un muscle ou à une partie mobile du corps.
